# FASCICULE DE BREVET EUROPEEN

(11) **EP 0 879 243 B1**
(45) Date de publication et mention de la délivrance du brevet: **30.05.2001**
(21) Numéro de dépôt: 97903422.0
(22) Date de dépôt: 07.02.1997
(51) Int. Cl.: C07K 5/06, A23L 1/236

(54) **ACIDE N-(3,3-DIMETHYLBUTYL)-L-ASPARTYL-D-ALPHA-AMINOALCANOIQUE N-(S)-1-PHENYL-1-ALCANAMIDE UTILE COMME AGENT EDULCORANT**
N-(3,3-DIMETHYLBUTYL)-L-ASPAREGINSÄURE-D-ALPHA-AMINOALKENSÄURE-1-PHENYL-1-ALKANAMIDE, ALS SÜSSTOFF VERWENDBAR
(N)-(S)-1-PHENYL-1-ALKANAMIDE (N)-(3,3-DIMETHYLBUTYL)-L-ASPARTYL-D-ALPHA AMINOALKANOIC ACID FOR USE AS A SWEETENER

(30) Priorité: 07.02.1996 FR 9601491
(43) Date de publication de la demande: 25.11.1998
(73) Titulaire: NOFRE, Claude, F-69003 Lyon (FR); TINTI, Jean-Marie, F-69680 Chassieu (FR)
(72) Inventeur: NOFRE, Claude, F-69003 Lyon (FR); TINTI, Jean-Marie, F-69680 Chassieu (FR)
(74) Mandataire: Hubert, Philippe
(86) Numéro de dépôt international: FR9700245
(87) Numéro de publication internationale: WO9729122

(56) Documents cités:
- WO-A-95/30689
- FR-A- 2 697 844
- FR-A- 2 719 592

## Description

La présente invention a pour objet de nouveaux composés, utiles comme agents édulcorants, dérivant de dipeptides, ainsi que leur procédé de préparation.

Ces nouveaux composés sont particulièrement utiles pour édulcorer des produits variés, en particulier les boissons, les aliments, les confiseries, les pâtisseries, les chewing-gums, les produits d'hygiène et les articles de toilette, ainsi que les produits cosmétiques, pharmaceutiques et vétérinaires.

On sait qu'un agent édulcorant, pour être utilisable à l'échelle industrielle, doit posséder, d'une part, un pouvoir sucrant intense, permettant de limiter le coût d'utilisation, et, d'autre part, une stabilité satisfaisante, c'est-à-dire compatible avec les conditions d'utilisation.

Parmi les agents édulcorants actuellement commercialisés, un dérivé dipeptidique, l'ester méthylique de la L-aspartyl-L-phénylalanine, connu sous le nom d'aspartame, de formule suivante: 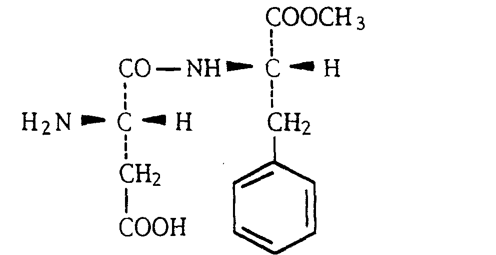 est le plus utilisé (U.S. 3,475,403). Le pouvoir sucrant, relativement faible, de ce composé est d'environ 120 à 180 fois celui du saccharose sur une base pondérale. Malgré d'excellentes qualités organoleptiques, ce composé a pour principal inconvénient d'être un produit cher, en raison de son intensité édulcorante relativement basse, et d'avoir une assez faible stabilité dans les conditions usuelles d'utilisation des édulcorants, ce qui limite fortement ses champs d'applications industrielles.

Dans le document FR 2697844 les Demandeurs ont proposé des agents édulcorants répondant à la formule générale suivante : 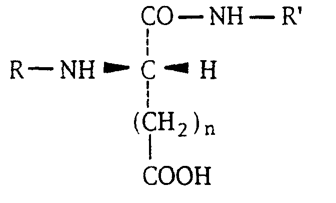 dans laquelle :
R est un groupe hydrocarboné ayant de quatre à treize atomes de carbone, saturé ou insaturé, acyclique, cyclique ou mixte ;
n est égal à 1 ou 2; et
R' est représenté par la formule suivante : 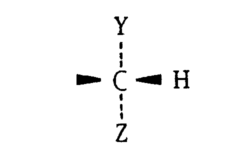 dans laquelle :
   Y est choisi parmi les groupes COOCH₃, COOC₂H₅, CH₃, CH₂OH, CON(CH₃)₂, C₆H₅, 2-furyle et H;
   Z est choisi parmi les groupes CH₂C₆H₅, C₆H₅, *n*-C₄H₉, COOCH₃, COOC₂H₅, COOC₃H₇, COOfenchyle, et CONHR" dans lequel R" est choisi parmi les groupes CH₃, CH₂CH₃, CH₂CH₂CH₃, CH₂CH₂CH(CH₃)₂, CH(CH₃)COOCH₃, CH(*c*-C₃H₅)₂, CH(*c*-C₃H₅)C(CH₃)₃, fenchyle, 2,6-diméthylcyclohexyle, 2,2,5,5-tétraméthylcyclopentyle, et 2,2,4,4-tétraméthyl-3-thiétanyle.

Une forme de réalisation décrite dans le document FR 2697844 couvre les dérivés de l'acide L-aspartique (n = 1) répondant à la formule suivante : 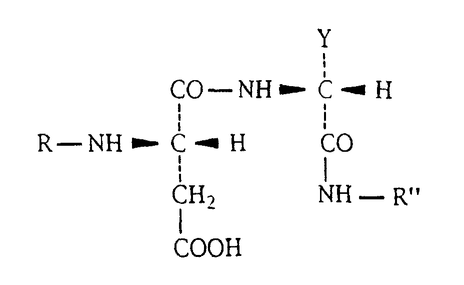 dans laquelle R, Y et R" sont tels que définis précédemment.

Un composé préféré dans cette forme de réalisation de l'invention est le *N*-(3,3-diméthylbutyl)-L-aspartyl-*N*-(dicyclopropylcarbinyl)-D-alaninamide de formule : 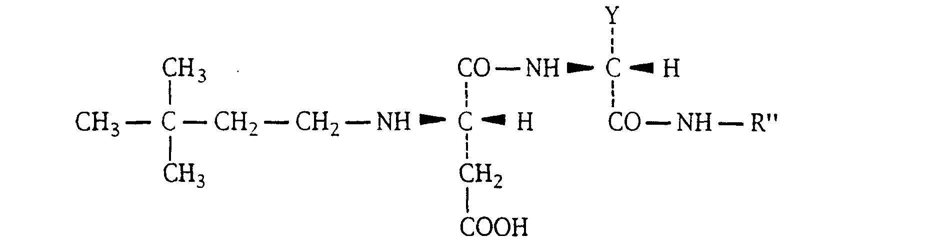 dans laquelle Y représente le groupe méthyle et R" représente le groupe dicyclopropylecarbinyle qui présente un pouvoir sucrant de 2 500 fois celui du saccharose sur une base pondérale par rapport à une solution de saccharose à 2 %.

Il a été observé, et ceci constitue le fondement de l'invention, qu'il était possible d'obtenir de nouveaux composés édulcorants répondant à la formule précitée pour des substituants Y et R" convenablement choisis, autres que ceux prévus dans ce document antérieur. Ainsi, lorsque Y représente un groupe éthyle, isopropyle ou (*R*)-α-hydroxyéthyle et R" un groupe (*S*)-α-éthylbenzyle, (*S*) -α-méthylbenzyle ou (*R*)-α-méthoxyméthylbenzyle, on obtient de nouveaux composés qui possèdent d'excellentes qualités organoleptiques associées à un pouvoir édulcorant très élevé, jusqu'à 8 000 fois le pouvoir sucrant du saccharose sur une base pondérale. Par ailleurs, la stabilité en solution acide ou neutre de ces composés est nettement plus élevée que celle de l'aspartame, ce qui devrait avoir pour effet d'élargir, par rapport à l'aspartame, leurs possibilités d'utilisation dans les préparations alimentaires.

La présente invention a donc pour objet de fournir de nouveaux agents édulcorants répondant à la formule suivante : 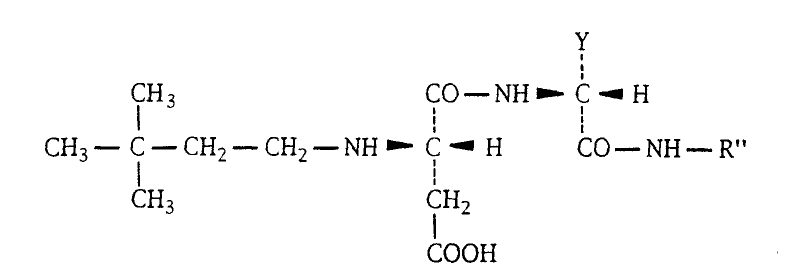 dans laquelle :
Y est C₂H₅, CH(CH₃)₂ ou (*R*) CH(OH)CH₃; et
R" est (*S*) CH(C₂H₅)C₆H₅, (*S*) CH(CH₃)C₆H₅ ou (*R*) CH(CH₂OCH₃)C₆H₅.

Les composés répondant à une forme de réalisation particulièrement avantageuse de l'invention sont l'acide *N*-(3,3-diméthylbutyl)-L-aspartyl-D-α-aminobutyrique *N*-(*S*)-1-phényl-1-propanamide de formule : 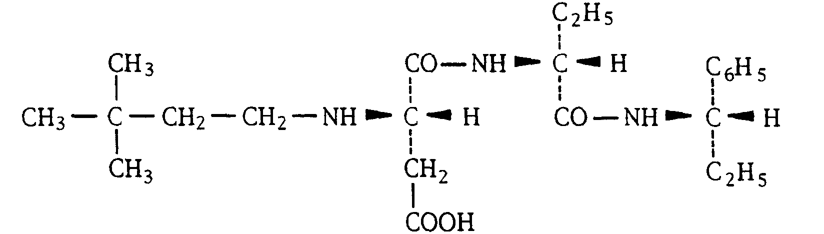 qui a un pouvoir sucrant d'environ 8 000 fois celui du saccharose sur une base pondérale par rapport à une solution de saccharose à 2 %;
le *N*-(3,3-diméthylbutyl)-L-aspartyl-D-valine *N*-(*S*)-1-phényl-1-propanamide de formule : 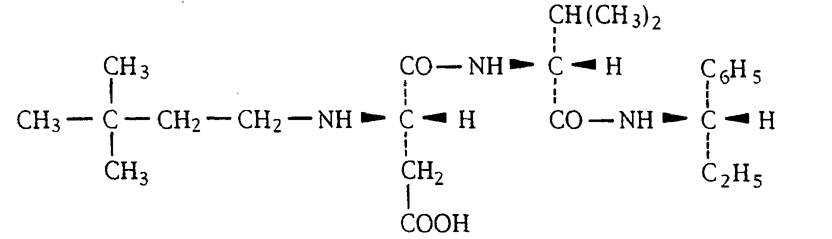 qui a un pouvoir sucrant d'environ 3 000 fois celui du saccharose sur une base pondérale par rapport à une solution de saccharose à 2 %;
1'acide *N*-(3,3-diméthylbutyl)-L-aspartyl-D-α-aminobu-tyrique *N*-(*S*)-1-phényl-2-méthoxy-1-éthanamide de formule : 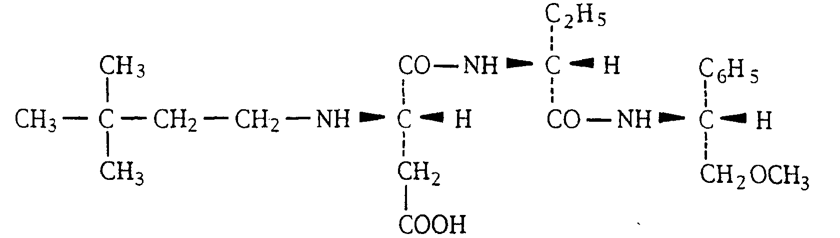 qui a un pouvoir sucrant d'environ 4 000 fois celui du saccharose sur une base pondérale par rapport à une solution de saccharose à 2 %, et
le *N*-(3,3-diméthylbutyl)-L-aspartyl-D-valine *N*-(*S*)-1-phényl-2-méthoxy-1-éthanamide de formule : 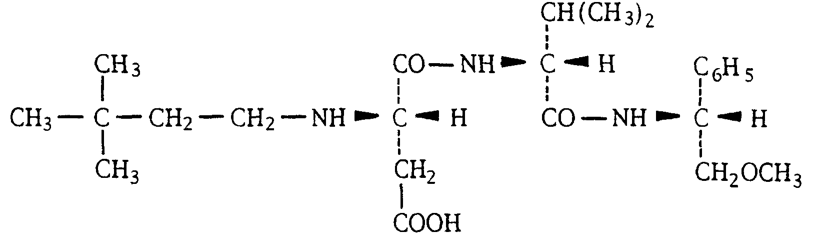 qui a un pouvoir sucrant d'environ 4 000 fois celui du saccharose sur une base pondérale par rapport à une solution de saccharose à 2 %.

Il a en outre été démontré que la stabilité de ces composés caractéristiques de l'invention est nettement plus élevée que celle de l'aspartame dans les conditions courantes d'utilisation pour les préparations alimentaires. Ceci est un avantage d'autant plus important que l'une des limites à l'utilisation de l'aspartame dans certaines préparations alimentaires provient de sa très faible stabilité en milieu proche de la neutralité, c'est-à-dire pour des pH voisins de 7, pH qui sont fréquemment rencontrés dans des produits tels que les produits laitiers, pâtisseries ou autres préparations qui nécessitent de surcroît une cuisson à haute température.

Il a également été démontré que la stabilité des composés de l'invention est par ailleurs améliorée en milieu acide à des pH voisins de 3, qui correspondent aux pH des boissons gazeuses qui constituent l'une des applications majeures des édulcorants.

Ainsi, par une étude de vieillissement accéléré par chauffage prolongé à 70 °C d'une solution aqueuse à pH 3, il a été montré que l'un des composés caractéristiques de l'invention, l'acide *N*-(3,3-diméthylbutyl)-L-aspartyl-D-α-aminobutyrique *N*-(*S*)-1-phényl-1-propanamide, présente une demi-vie d'environ 72 heures. A titre de comparaison, la demi-vie de l'aspartame dans ces mêmes conditions n'est que d'environ 24 heures, ce qui correspond à une stabilité environ 3 fois plus élevée pour le composé selon l'invention.

Une même étude de vieillissement accéléré à pH 7 a montré que ce même composé, l'acide *N*-(3,3-diméthylbutyl)-L-aspartyl-D-α-aminobutyrique *N*-(*S*)-1-phényl-1-propanamide, présente une demi-vie d'environ 12 jours, alors que la demi-vie de l'aspartame dans les mêmes conditions n'est que de 10 minutes, ce qui correspond à une stabilité environ 1700 fois plus élevée pour le composé selon l'invention. Des résultats comparables ont été obtenus pour les autres composés caractéristiques de l'invention.

Du fait de leur pouvoir sucrant élevé, un autre avantage des composés de l'invention comparativement à l'aspartame est de permettre, dans leur application aux produits alimentaires, l'utilisation de quantités très faibles d'agent actif. C'est ainsi par exemple qu'il est possible de remplacer, dans un litre de boisson gazeuse, 550 mg d'aspartame par environ 20 mg de l'acide *N*-(3,3-diméthylbutyl)-L-aspartyl-D-α-aminobutyrique *N*-(*S*)-1-phényl-1-propanamide de l'invention, et de diminuer ainsi d'environ 27 fois les quantités d'édulcorant consommées, tout en maintenant des qualités organoleptiques identiques.

Les agents édulcorants de la présente invention peuvent être ajoutés à tout produit comestible dans lequel on désire apporter un goût sucré, à condition de les ajouter en proportions suffisantes pour atteindre le niveau de sucrosité désiré. La concentration optimale d'utilisation de l'agent édulcorant dépendra de facteurs divers tels que, par exemple, le pouvoir sucrant de l'agent édulcorant, les conditions de stockage et d'utilisation des produits, les constituants particuliers des produits et le niveau de sucrosité désiré. Toute personne qualifiée peut facilement déterminer la proportion optimale d'agent édulcorant qui doit être employée pour l'obtention d'un produit comestible en réalisant des analyses sensorielles de routine. Les agents édulcorants de la présente invention seront, en général, ajoutés aux produits comestibles dans des proportions allant, suivant le pouvoir édulcorant du composé, de 5 mg à 50 mg d'agent édulcorant par kilogramme ou par litre de produit comestible. Les produits concentrés contiendront évidemment des quantités plus élevées d'agent édulcorant, et seront ensuite dilués suivant les intentions finales d'utilisation.

Les agents édulcorants de la présente invention peuvent être ajoutés sous forme pure aux produits à édulcorer, mais, en raison de leur pouvoir sucrant élevé, ils sont généralement mélangés à un support ("carrier") ou à un agent de charge ("bulking agent") approprié.

Avantageusement, les supports ou agents de charge appropriés sont choisis dans le groupe constitué par le polydextrose, l'amidon, les maltodextrines, la cellulose, la méthylcellulose, la carboxyméthylcellulose et autres dérivés de la cellulose, l'alginate de sodium, les pectines, les gommes, le lactose, le maltose, le glucose, la leucine, le glycérol, le mannitol, le sorbitol, le bicarbonate de sodium, les acides phosphorique, citrique, tartrique, fumarique, benzoïque, sorbique, propionique, et leurs sels de sodium, potassium et calcium, et leurs équivalents.

Les agents édulcorants conformes à l'invention peuvent, dans un produit comestible, être employés seuls, comme unique agent édulcorant, ou en combinaison avec d'autres agents édulcorants tels que le saccharose, le sirop de maïs, le fructose, les dérivés ou analogues dipeptidiques sucrés (aspartame, alitame), la néohespéridine dihydrochalcone, l'isomaltulose hydrogéné, le stévioside, les sucres L, la glycyrrhizine, le xylitol, le sorbitol, le mannitol, l'acésulfame-K, la saccharine et ses sels de sodium, potassium, ammonium et calcium, l'acide cyclamique et ses sels de sodium, potassium et calcium, le sucralose, la monelline, la thaumatine, et leurs équivalents.

Les composés de la présente invention sont préparés par une N-alkylation réductive consistant à condenser le précurseur dipeptidique de formule : 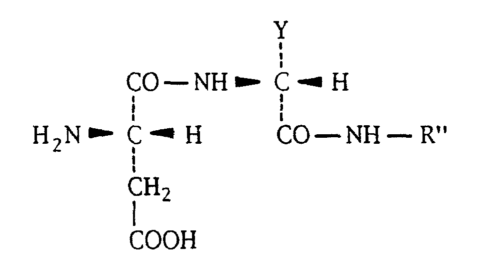 dans laquelle Y et R" sont tels que définis précédemment, avec le 3,3-diméthylbutyraldéhyde en présence d'un agent de réduction. Cet agent de réduction est soit l'hydrogène sous une pression relative comprise entre 1 et 3 bars en présence d'un catalyseur à base de platine ou de palladium suivant le procédé décrit dans le document WO 95/30689, soit le cyanoborohydrure de sodium suivant le procédé décrit dans le document FR 92 13615.

Le précurseur dipeptidique de formule ci-dessus peut être obtenu facilement par la mise en oeuvre des principes de base de la synthèse peptidique : protection des groupes amino et carboxyle des amino acides précurseurs et leur déblocage, et méthodes classiques d'activation et de couplage peptidique.

Ces techniques sont décrites de façon détaillée dans de nombreuses publications, parmi lesquelles on peut citer tout particulièrement M. Bodanszky et A. Bodanszky, The Practice of Peptide Synthesis, Springer-Verlag, New York, 1984, 284 pp.

La purification des composés de l'invention est réalisée selon les techniques standards telles que la recristallisation ou la chromatographie. Leur structure et leur pureté ont été contrôlées par les techniques classiques (chromatographie sur couche mince, chromatographie liquide haute performance, spectrométrie infrarouge, résonance magnétique nucléaire, analyse élémentaire).

Le pouvoir édulcorant des composés décrits dans les exemples a été évalué par un groupe de huit personnes expérimentées. Pour cela, les composés, en solution aqueuse à des concentrations variables, sont comparés, sur le plan gustatif, à une solution témoin de saccharose à 2 %, à 5 % ou à 10 %. Le pouvoir édulcorant du composé, testé par rapport au saccharose, correspond alors au rapport pondéral qui existe entre le composé et le saccharose à égale intensité édulcorante, c'est-à-dire quand les saveurs sucrées de la solution du composé testé et de la solution témoin de saccharose sont considérées, par une majorité de personnes, avoir la même intensité édulcorante.

La stabilité des composés de l'invention et de l'aspartame a été mesurée en dosant, par chromatographie liquide haute performance (HPLC), la quantité de produit restant après un vieillissement accéléré en milieu acide (tampon phosphate à pH 3) ou en milieu neutre (tampon phosphate à pH 7) et à la température de 70 °C. La stabilité du composé ainsi testé est évaluée par sa demi-vie (temps correspondant à 50 % de dégradation).

La manière dont l'invention peut être réalisée et les avantages qui en découlent ressortiront mieux des exemples de réalisation qui suivent.

### EXEMPLES

### Préparation de l'acide N-(3,3-diméthylbutyl)-L-aspartyl-D-α-aminobutyrique N-(S)-1-phényl-1-propanamide de formule :

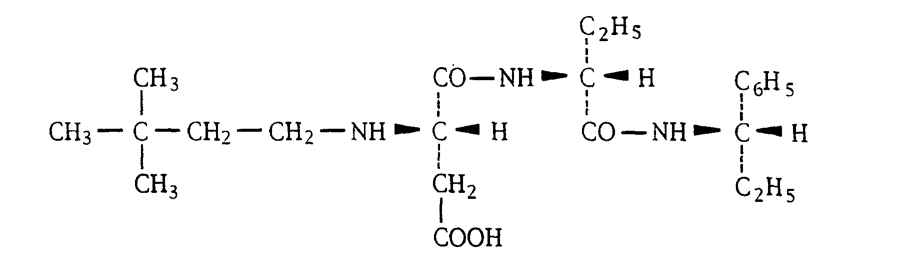

Un mélange de 15 g (0,145 mole) d'acide D-α-aminobutyrique (produit Aldrich n° 11,612-2) et de 5,82 g (0,145 mole) d'hydroxyde de sodium dans 150 cm³ d'eau est refroidie à 0 °C. A cette solution, sont simultanément ajoutés, goutte à goutte, 24,38 g (0,145 mole) de chloroformiate de benzyle et 5,82 g (0,145 mole) d'hydroxyde de sodium en solution aqueuse 4N. L'agitation est ensuite maintenue durant 3 h à 0 °C. Le mélange réactionnel est lavé par l'éther éthylique (3 x 30 cm³) puis est acidifié par une solution d'acide chlorhydrique 6N jusqu'à l'obtention d'un pH d'environ 1. Le précipité blanc formé est séparé par filtration, lavé par l'eau puis séché. On obtient finalement 26 g (rendement 75 %) d'acide *N*-benzyloxycarbonyl-D-α-aminobutyrique dont le point de fusion est de 80 °C.

A une solution de 3 g (13 mmoles) du composé ainsi préparé dans 50 cm³ de tétrahydrofurane refroidie à15 °C sont successivement ajoutés 1,28 g (13 mmoles) de N-méthylmorpholine et 1,15 g (13 mmoles) de chloroformiate d'isobutyle. Après 2 minutes d'agitation à cette température, est ajouté 1,71 g (13 mmoles) de (*S*)-1-phényl-1-propanamine (préalablement préparé selon le document *J*. *Chem*. *Soc.,* 1940, pp. 336-8). Le mélange réactionnel est lentement réchauffé puis est agité durant 2 heures à la température ambiante. Le précipité de chlorhydrate de N-méthylmorpholine est éliminé par filtration, puis est lavé par 20 cm³ de tétrahydrofurane. Les filtrats sont concentrés à sec sous vide et le résidu obtenu est repris dans 150 cm³ d'éther éthylique. La solution obtenue est lavée successivement par une solution d'acide chlorhydrique 0,1 N, une solution de carbonate de sodium à 5 % puis par l'eau (3 x 30 cm³ pour chaque lavage). Après séchage de la solution éthérée sur sulfate de sodium anhydre, la solution est concentrée à sec sous vide, ce qui conduit à 3,8 g d'un résidu solide blanc, l'acide *N*-benzyloxycarbonyl-D-α-aminobutyrique *N*-(*S*)-1-phényl-1-propanamide (rendement 82 %) dont le point de fusion est de 129 °C. Sa pureté est contrôlée par chromatographie sur couche mince sur gel de silice G 60 (support silice Merck n° 1.05554), éluant chloroforme-acétone (9-1), révélation par le mélange dichromate de potassium-acide sulfurique concentré, Rf = 0,45.

Une solution de 3,6 g (10 mmoles) de l'acide *N*-benzyloxycarbonyl-D-α-aminobutyrique *N*-(*S*)-1-phényl-1-propanamide dans 100 cm³ de méthanol est soumise, en présence de 350 mg de palladium à 10 % sur charbon actif (produit Fluka n° 75990), à l'hydrogène sous pression atmosphérique durant 18 h. Après élimination du catalyseur par filtration, la solution est concentrée à sec sous vide. On obtient ainsi 2,1 g (rendement 91 %) d'acide D-α-aminobutyrique *N*-(*S*)-1-phényl-l-propanamide, sous forme d'un résidu huileux. Sa pureté est contrôlée par chromatographie sur couche mince sur gel de silice G 60 (support silice Merck n° 1.05554), éluant butanol-acide acétique-eau (8-2-2), révélation à la ninhydrine, Rf = 0,57.

A une solution de 3,58 g (10 mmoles) d'acide *N*-benzyloxycarbonyl-L-aspartique β-benzyl ester (produit Bachem n° C-1350) dans 50 cm³ de tétrahydrofurane refroidie à -15 °C sont successivement ajoutés 1 g (10 mmoles) de N-méthylmorpholine et 1,37 g (10 mmoles) de chloroformiate d'isobutyle. Après 2 minutes d'agitation à cette température, sont ajoutés 2,1 g (10 mmoles) d'acide D-α-aminobutyrique *N*-(*S*)-1-phényl-1-propanamide précédemment préparé. Le mélange réactionnel est lentement réchauffé puis est agité durant 2 heures à la température ambiante. Le précipité de chlorhydrate de N-méthylmorpholine est éliminé par filtration, puis est lavé par 20 cm³ de tétrahydrofurane. Les filtrats sont concentrés à sec sous vide et le résidu obtenu est trituré dans 50 cm³ d'éther éthylique. Le solide blanc formé est séparé par filtration puis est à nouveau lavé par 20 cm³ d'éther éthylique. On obtient ainsi 5 g (rendement 89 %) d'acide *N*-benzyloxycarbonyl-β-benzylester-L-aspartyl-D-α-aminobutyrique *N*-(*S*)-1-phényl-1-propanamide dont le point de fusion est de 130 °C. Sa pureté est contrôlée par chromatographie sur couche mince sur gel de silice G 60 (support silice Merck n° 1.05554), éluant chloroforme-acétone (8-2), révélation par le mélange sulfochromique, Rf = 0,64.

Une solution de 5 g (8,9 mmoles) d'acide *N*-benzyloxycarbonyl-β-benzylester-L-aspartyl-D-α-aminobutyrique *N*-(*S*)-1-phényl-1-propanamide dans 100 cm³ de méthanol est soumise, en présence de 500 mg de palladium à 10 % sur charbon actif (produit Fluka n° 75990), à l'hydrogène sous pression atmosphérique durant 18 h. Après élimination du catalyseur par filtration, la solution est concentrée à sec sous vide. On obtient ainsi 2,4 g (rendement 80 %) d'acide L-aspartyl-D-α-aminobutyrique *N*-(*S*)-1-phényl-1-propanamide, sous forme d'un solide blanc. Sa pureté est contrôlée par chromatographie sur couche mince sur gel de silice G 60 (support silice Merck n° 1.05554), éluant butanol-acide acétique-eau (8-2-2), révélation à la ninhydrine, Rf = 0,50. Son point de fusion dans cet état amorphe est de 195 °C.

Dans un réacteur équipé d'une agitation permettant d'assurer un très bon transfert de l'hydrogène gazeux dans la phase liquide, on introduit sous agitation et dans l'ordre : 15 cm³ d'une solution aqueuse d'acide acétique 0,1 M, 26 mg de palladium sur charbon actif (produit Fluka n° 75990), 58 mg (0,57 mmole) de 3,3-diméthylbutyraldéhyde d'origine commerciale (Aldrich n° 35,990-4), 15 cm³ de méthanol et 130 mg (0,38 mmole) d'acide L-aspartyl-D-α-aminobutyrique *N*-(*S*)-1-phényl-1-propanamide précédemment préparé.

Après avoir purgé le réacteur par un courant d'azote, le mélange est soumis à une hydrogénation à la pression relative d'environ 1-2 bars (0,1-0,2 MPa) et à température ambiante durant 18 heures, puis à nouveau durant 8 heures après une nouvelle addition de 19 mg (0,19 mmole) de 3,3-diméthylbutyraldéhyde. L'avancement de la réaction est contrôlé par chromatographie sur couche mince (CCM) sur support de Gel de silice 60 F254 sur feuilles d'aluminium **(Merck n° 1.05554), éluant :** butanol-acide acétique-eau (8:2:2), révélation à la ninhydrine : Rf 0,67.

La réaction est finalement interrompue en purgeant le réacteur par un courant d'azote et en séparant le catalyseur par filtration sur filtre fin (0,5 µm). La solution est alors concentrée par évaporation sous vide et le solide blanc obtenu est lavé par environ 50 cm³ d'éther éthylique. On obtient finalement 150 mg d'acide *N*-(3,3-diméthylbutyl)-L-aspartyl-D-α-aminobutyrique *N*-(*S*)-1-phényl-1-propanamide (rendement 92 %) sous la forme d'une poudre blanche de grande pureté (supérieure à 98 % par H.P.L.C.) et dont le point de fusion est de 167 °C.
Formule moléculaire : C₂₃H₃₇N₃O₄
RMN (200 MHz, 1H, ppm), DMSO D6 : 0,80 (s, 15 H), 1,33 (t, 2H), 1,67 (m, 4H), 2,4 (m, 4H), 3,54 (m, 1H), 4,30 (q, 1H), 4,70 (q, 1H), 7,22 (m, 1H), 7,28 (s, 5H), 8,35 (t, 2H).
Chromatographie liquide haute performance sur colonne Merck de type "Lichrospher 100 RP-18 endcapped", longueur 244 mm, diamètre 4,6 mm, éluant : tampon acétate d'ammonium 65 mM - acétonitrile (70:30), débit de 1 ml/min, détecteur : réfractomètre, temps de rétention 16,1 min.

Le pouvoir édulcorant de ce composé correspond approximativement, sur une base pondérale, à 8 000 fois celui du saccharose par comparaison avec une solution de saccharose à 2 %, à 6 000 fois celui du saccharose par comparaison avec une solution de saccharose à 5 % et à 5 000 fois celui du saccharose par comparaison avec une solution de saccharose à 10 %.

Par comparaison avec l'aspartame, une solution aqueuse de 20 mg/L de ce composé est équivalente en intensité édulcorante à une solution de 550 mg/L d'aspartame, ce qui correspond à un pouvoir sucrant environ 27 fois plus élevé que celui de l'aspartame.

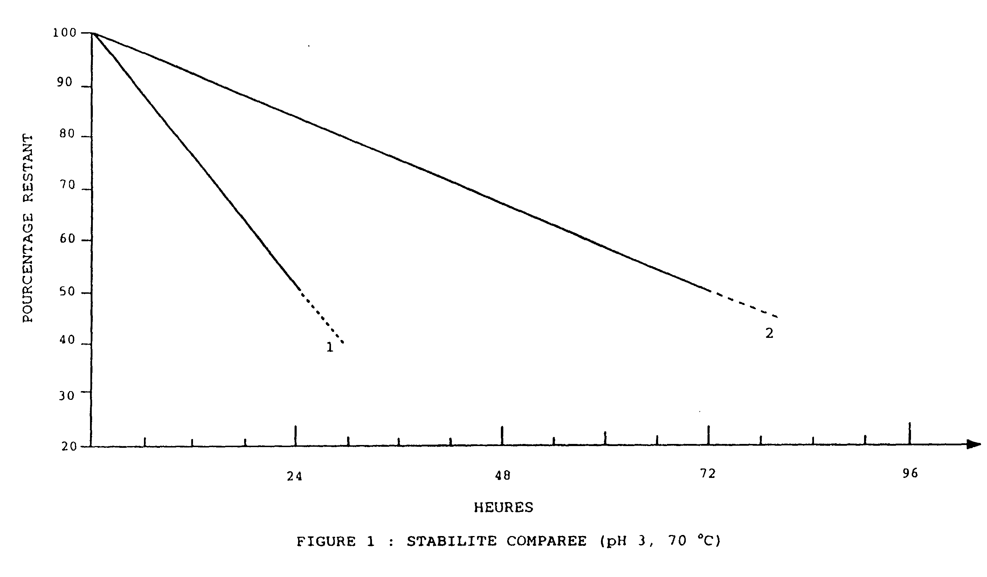

Dans la figure 1 annexée, est donné un diagramme comparatif des courbes de stabilité de l'aspartame (courbe 1) et de l'acide *N*-(3,3-diméthylbutyl)-L-aspartyl-D-α-aminobutyrique *N*-(*S*)-1-phényl-1-propanamide de la présente invention (courbe 2) obtenues au cours d'un vieillissement accéléré par chauffage à 70 °C de leurs solutions en milieu acide de pH 3 (tampon phosphate). Dans ces conditions, la demi-vie de l'aspartame est de 24 heures, alors que la demi-vie du composé de l'invention est d'environ 72 heures, ce qui correspond à une stabilité qui est 3 fois plus élevée en faveur du composé de l'invention par rapport à l'aspartame.

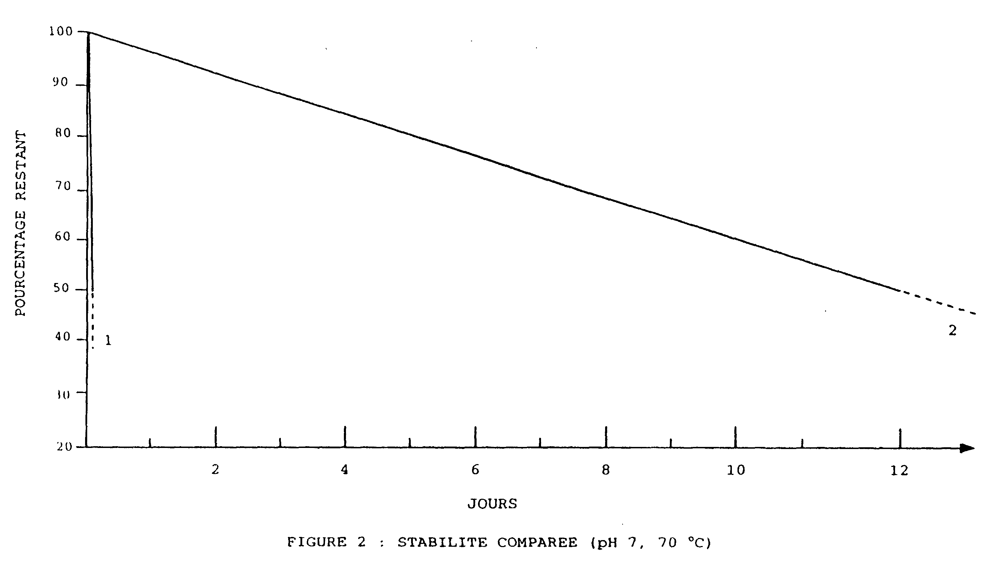

Dans la figure 2 annexée, est donné un diagramme comparatif des courbes de stabilité de l'aspartame (courbe 1) et de l'acide N-(3,3-diméthylbutyl)-L-aspartyl-D-α-aminobutyrique N-(*S*)-1-phényl-1-propanamide de l'invention (courbe 2) obtenues au cours d'un vieillissement accéléré par chauffage à 70 °C de leurs solutions en milieu neutre de pH 7. Dans ces conditions, l'aspartame est très peu stable (demi-vie de 10 minutes) alors que le composé de l'invention présente une demi-vie d'environ 12 jours, ce qui correspond à une stabilité qui est environ 1700 fois plus élevée que celle de l'aspartame.

Le pouvoir sucrant d'autres composés selon l'invention, obtenus suivant un protocole expérimental similaire à celui décrit ci-dessus et que l'homme de l'art retrouvera facilement, est donné dans le Tableau 1.

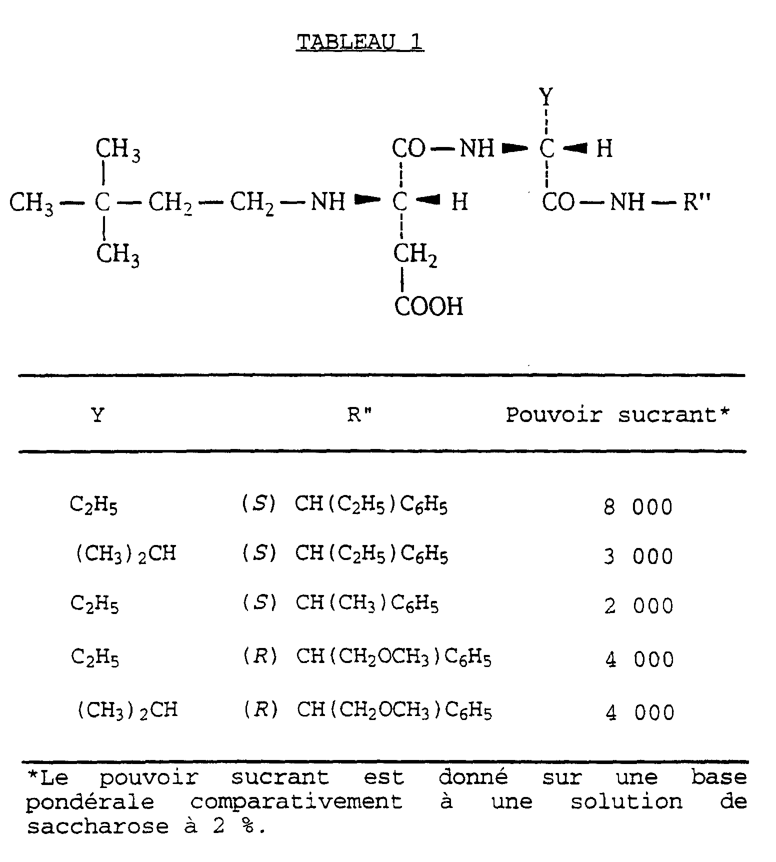

## Revendications

1. Composés de formule : 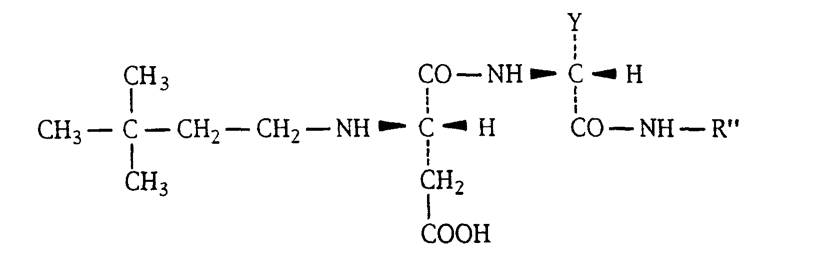 dans laquelle :
Y est C₂H₅, CH(CH₃)₂ ou (R) CH(OH)CH₃; et
R" est (S) CH(C₂H₅)C₆H₅, (*S*) CH(CH₃)C₆H₅ ou (*R*) CH(CH₂OCH₃)C₆H₅.

2. Composé selon la revendication 1 caractérisé en ce qu'il s'agit de l'acide *N*-(3,3-diméthylbutyl)-L-aspartyl-D-α-aminobutyrique *N*-(*S*)-1-phényl-1-propanamide de formule :

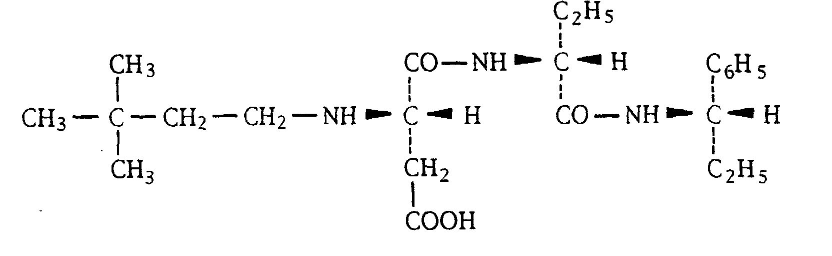

3. Composé selon la revendication 1 caractérisé en ce qu'il s'agit du *N*-(3,3-diméthylbutyl)-L-aspartyl-D-valine *N*-(*S*)-1-phényl-1-propanamide de formule :

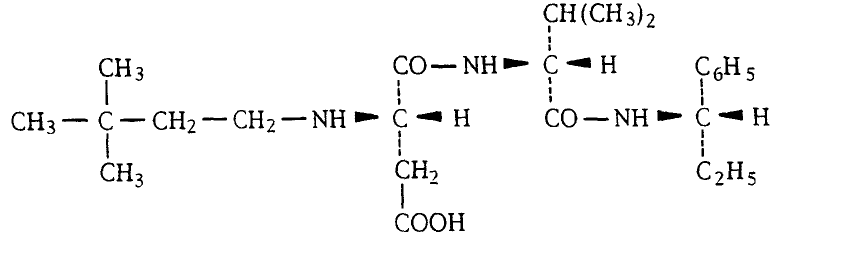

4. Composé selon la revendication 1 caractérisé en ce qu'il s'agit de l'acide *N*-(3,3-diméthylbutyl)-L-aspartyl-D-α-aminobutyrique *N*-(*S*)-1-phényl-2-méthoxy-1-éthanamide de formule :

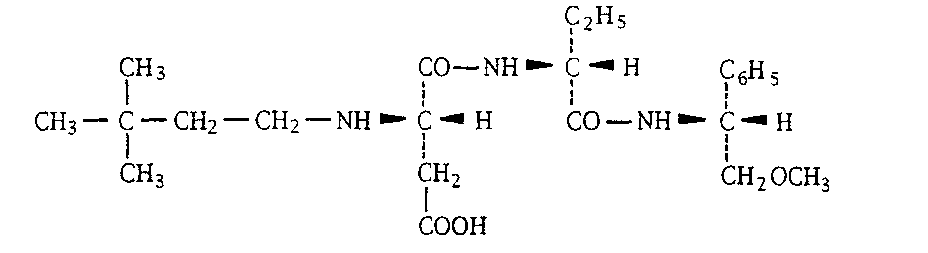

5. Composé selon la revendication 1 caractérisé en ce qu'il s'agit du *N*-(3,3-diméthylbutyl)-L-aspartyl-D-valine *N*-(*S*)-1-phényl-2-méthoxy-1-éthanamide de formule :

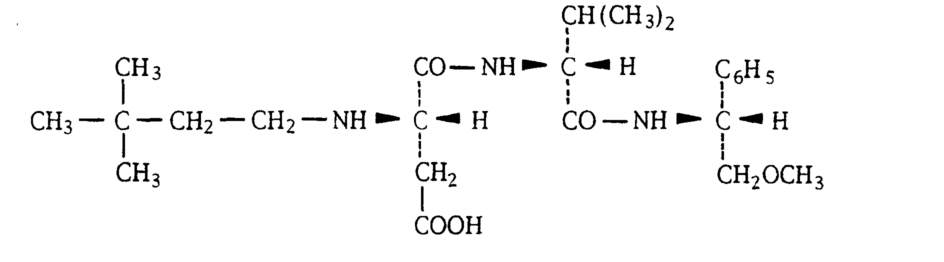

6. Application des composés selon l'une quelconque des revendications 1 à 5 comme agents édulcorants.

7. Procédé de préparation des composés selon l'une quelconque des revendications 1 à 5 caractérisé en ce qu'il consiste à traiter par un agent de réduction un mélange de 3,3-diméthylbutyraldéhyde et d'un composé de formule : 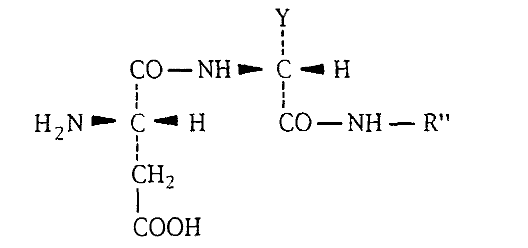 dans laquelle Y et R" sont tels que définis dans la revendication 1.

8. Procédé selon la revendication 7 caractérisé en ce que l'agent de réduction est l'hydrogène à une pression relative comprise entre 1 et 3 bars en présence d'un catalyseur à base de platine ou de palladium.

9. Procédé selon la revendication 7 caractérisé en ce que l'agent de réduction est le cyanoborohydrure de sodium.

## Patentansprüche

1. Verbindungen der Formel: 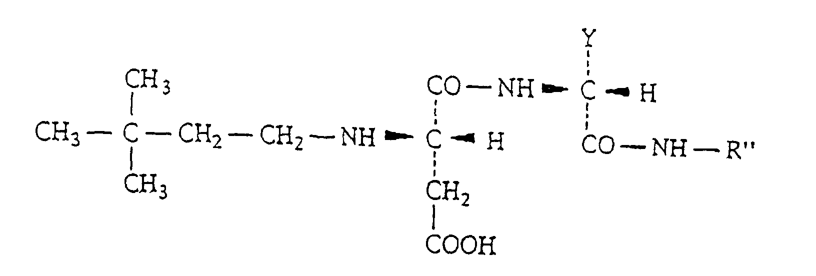 in der:
Y C₂H₅, CH(CH₃)₂ oder (*R*) CH(OH)CH₃ ist und
R'' (*S*) CH(C₂H₅)C₆H₅, (*S*) CH(CH₃)C₆H₅ oder (*R*) CH(CH₂OCH₃)C₆H₅ ist.

2. Verbindung nach Anspruch 1, dadurch gekennzeichnet, dass es sich um das N-(3,3-Dimethylbutyl)-L-aspartyl-D-α-aminobuttersäure-N-(*S*)-1-phenyl-l-propanamid der Formel: 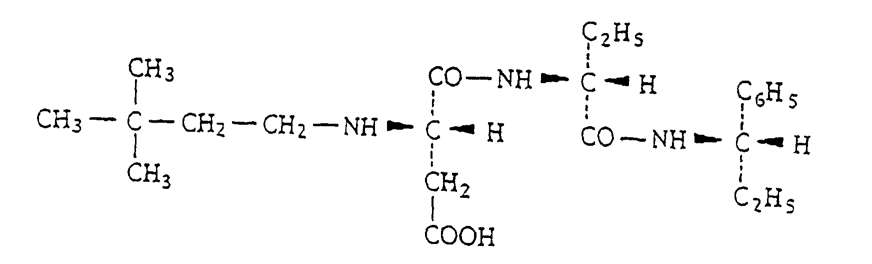 handelt.

3. Verbindung nach Anspruch 1, dadurch gekennzeichnet, dass es sich um das N-(3,3-Dimethylbutyl)-L-aspartyl-D-valin-N-(*S*)-1-phenyl-1-propanamid der Formel: 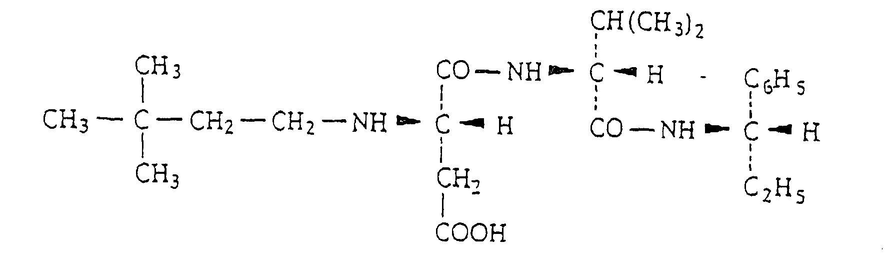 handelt.

4. Verbindung nach Anspruch 1, dadurch gekennzeichnet, dass es sich um das N-(3,3-Dimethylbutyl)-L-aspartyl-D-α-aminobuttersäure-N-(*S*)-1-phenyl-2-methoxy-1-ethanamid der Formel: 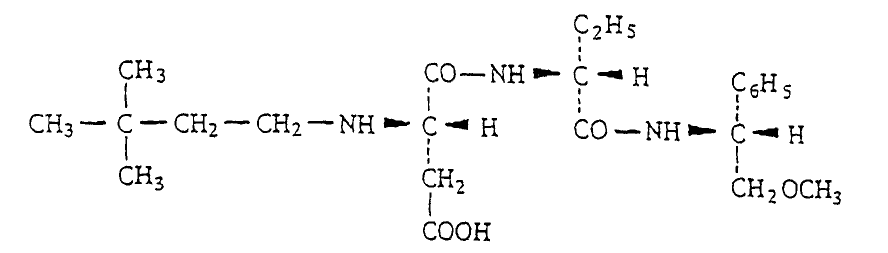 handelt.

5. Verbindung nach Anspruch 1, dadurch gekennzeichnet, dass es sich um das N-(3,3-Dimethylbutyl)-L-aspartyl-D-valin-N-(*S*)-1-phenyl-2-methoxy-1-ethanamid der Formel: 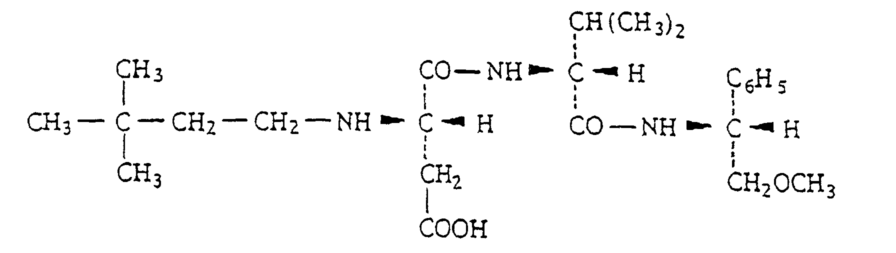 handelt.

6. Anwendung der Verbindungen nach einem der Ansprüche 1 bis 5 als Süßungsmittel.

7. Verfahren zur Herstellung der Verbindungen nach einem der Ansprüche 1 bis 5, dadurch gekennzeichnet, dass es darin besteht, eine Mischung von 3,3-Dimethylbutyraldehyd und einer Verbindung der Formel: 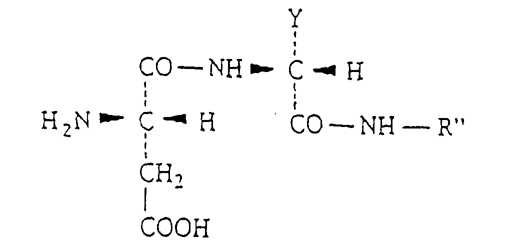 in der Y und R'' wie in Anspruch 1 definiert sind, mittels eines Reduktionsmittels zu behandeln.

8. Verfahren nach Anspruch 7, dadurch gekennzeichnet, dass das Reduktionsmittel Wasserstoff bei einem relativen Druck zwischen 1 und 3 bar in Gegenwart eines Katalysators auf Basis von Platin oder Palladium ist.

9. Verfahren nach Anspruch 7, dadurch gekennzeichnet, dass das Reduktionsmittel Natriumcyanborhydrid ist.

## Claims

1. Compounds of formula: 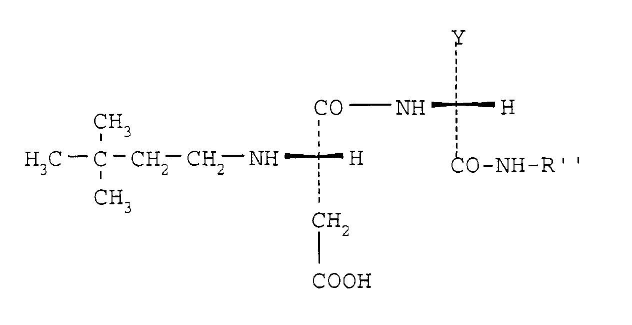 in which:
Y is C₂H₅, CH(CH₃)₂ or (*R*) CH(OH)CH₃; and
R" is (*S*) CH(C₂H₅)C₆H₅, (*S*) CH(CH₃)C₆H₅ or (*R*) CH(CH₂OCH₃)C₆H₅.

2. The compound according to claim 1 characterised in that it is *N*-(3,3-dimethylbutyl)-L-aspartyl-D-α-aminobutyric acid *N*-(*S*)-1-phenyl-1-propanamide of formula:

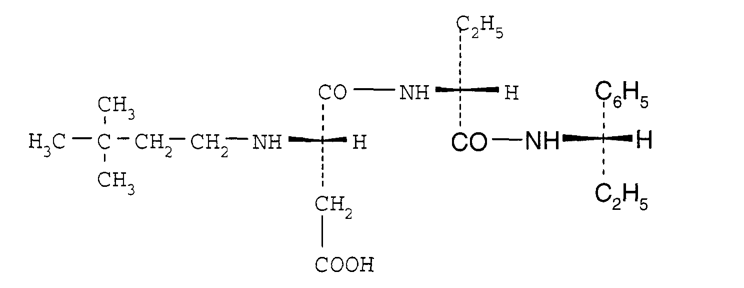

3. The compound according to claim 1 characterised in that it is *N*-(3,3-dimethylbutyl)-L-aspartyl-D-valine *N-*(*S*)-1-phenyl-1-propanamide of formula:

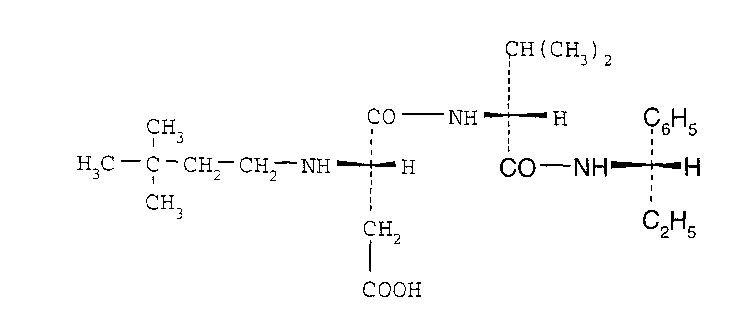

4. The compound according to claim 1 characterised in that it is *N*-(3,3-dimethylbutyl)-L-aspartyl-D-α-aminobutyric acid *N*-(*S*)-1-phenyl-2-methoxy-1-ethanamide of formula:

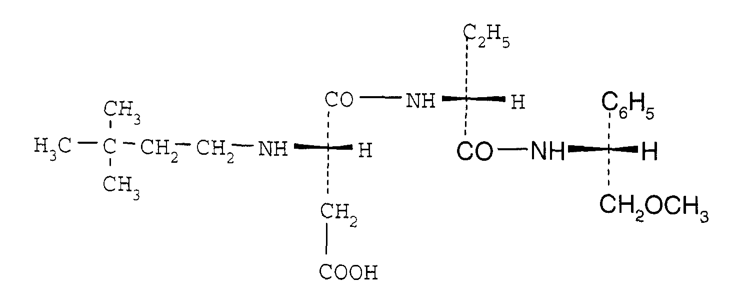

5. The compound according to claim 1 characterised in that it is *N*-(3,3-dimethylbutyl)-L-aspartyl-D-valine *N*-(*S)*-1-phenyl-2-methoxy-1-ethanamide of formula:

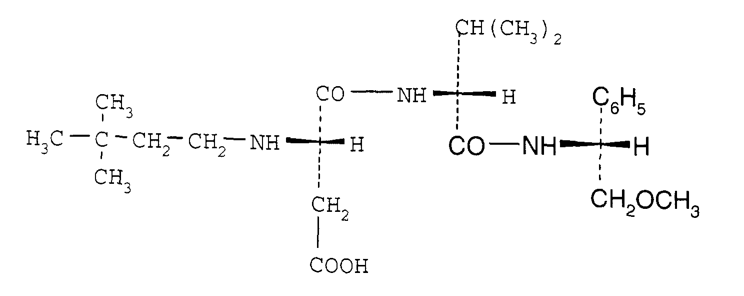

6. The application of the compounds according to any one of claims 1 to 5 as sweetening agents.

7. A method of preparation of the compounds according to any one of claims 1 to 5, characterised in that it consists in the treatment, by a reducing agent, of a mixture of 3,3-dimethylbutyraldehyde and a compound of formula: 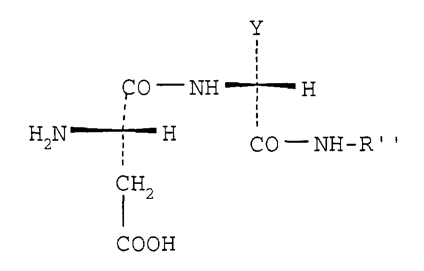 in which Y and R" are as defined in claim 1.

8. The process according to claim 7, characterised in that the reducing agent is hydrogen at a relative pressure between 1 and 3 bars in the presence of a catalyst based on platinum or palladium.

9. The process according to claim 7, characterised in that the reducing agent is sodium cyanoborohydride.
